# EUROPEAN PATENT APPLICATION

(11) **EP 2 116 256 A1**
(43) Date of publication of application: **11.11.2009**
(21) Application number: 08712105.9
(22) Date of filing: 28.02.2008
(51) Int. Cl.: A61K 39/02, A61K 9/70, A61K 31/7088, A61K 39/00, A61K 39/002, A61K 39/12, A61K 39/35, A61K 39/39, A61K 47/10, A61K 47/14, A61K 47/32, A61K 47/36, A61P 37/04, A61P 43/00

(54) **TRANSDERMAL IMMUNE PREPARATION, METHOD FOR PRODUCTION OF THE SAME, AND TRANSDERMAL IMMUNIZATION METHOD USING THE SAME**

(30) Priority: 01.03.2007 JP 2007089368
(71) Applicant: Cosmed Pharmaceutical Co., Ltd., Kamigyo-ku Kyoto-shi Kyoto 602-0841 (JP); Osaka University, Suita-shi Osaka 565-0871 (JP)
(72) Inventor: NAKAGAWA, Shinsaku, Suita-shi Osaka 565-0871 (JP); OKADA, Naoki, Suita-shi Osaka 565-0871 (JP); FUJITA, Takuya, Kyoto-shi Kyoto 607-8414 (JP); KAMIYAMA, Fumio, Kyoto-shi Kyoto 602-0841 (JP); QUAN, Ying-shu, Kyoto-shi Kyoto 602-0841 (JP)
(74) Representative: Merkle, Gebhard
(86) International application number: PCT/JP2008/053534
(87) International publication number: WO 2008/105504

(57) **Abstract**

This invention is concernced with transdermal immune preparations which can exhibit a high immunological activity without containing any adjuvant, methods for producing the preparation, and transdermal immunization methods using the preparation.

The transdermal immune preparations can regulate the immune response by applying the preparation to an intact skin, and which comprises a base material and an antigen or artificial synthetic nucleic acid laminated on the surface of the base material; the methods for producing transdermal immune preparations which comprises putting drops of an aqueous solution or suspension prepared by dissolving or suspending an antigen in water or a water-containing alcohol on the surface of a base material in order for the water or the water-containing alcohol to be absorbed in the base material, thereby forming an layer containing the antigen in a concentrated state on the surface of the base material; and the transdermal immunization methods which is haracterized by applying the transdermal immune preparation to an intact skin to regulate the immune response.

## Description

### Field of invention

The present invention relates to a transdermal immune preparation which can regulate the immune response, method for producing the preparation, and transdermal immunization method using the preparation.

### Background of the invention

For transdermal immunization, both the permeation of antigen through skin (usually antigens are not permeable) and immune response to antigen are necessary. Usually, antigens are macromolecules with a molecular weight higher than 1000 Dalton, which makes it difficult to permeate through the skin. Therefore, in order to put transdermal immunization into practice, the stratum corneum of the skin has to be stripped, and then an antigen-containing preparation is applyed to the stratum corneum stripped skin (See Patent reference 1). However, stripping of the stratum corneum not only makes the administration of antigen much troublesome, but also makes it easy for the stripped site to be infected by bacteria.

Therefore, the studies of transdermal immune preparation which the transdermal immunization can be achieved by applying the preparation to an intact skin have been carried out. For example, a transdermal immune preparation which contains more than one antigen and adjuvant components was proposed in Patent reference 2. In this patent, at lease one of said component is dry type and can be applied to intact skin. The preparation which can induce specific immune response to antigen and the method for producing dry transdermal immune preparation were introduced as follows: (a) at least one immunological active ingredient was provided (in this preparation, at least one immunological active ingredient which has antigen activity); (b) preparation of immunological active liquid by dissolving at least one immunological active ingredient; (c) drying of immunological active liquid on surface of solid base; (d) the producing of the preparation by mixing the immune active components. Here, the preparation is applicable to the skin and at least one of the active components is dry type.

Adjuvant can activate and/or induce antigen presenting cell of immune system (such as epidermal langerhans cells, dendritic cells, macrophage, B lymphocytes etc.) and ingest the antigen. However, adjuvant is a substance which can result in inflammation. It can not only lead to skin inflammation, but also destroy the immune balance, thereby adjuvant not being desirable to be administrated to human body. Additionally, because antigen in above preparation was dispersed in matrix of gauze etc, or dissolved in liquid, the skin contact concentration of antigen was diluted and the immune activity was low.
[Patent reference 1] Japanese Paten Laid-Open No. 10-316585
[Patent reference 2] Japanese Paten Laid-Open No. 2003-523937

### Description of the invention

The object of this invention is to provide a transdermal immune preparation which overcomes the above deficiencies without containing any adjuvant, and to provide a method for producing the preparation; and a transdermal immunization method using the preparation.
The transdermal immune preparation in this invention is a preparation which can regulate the immune response by applying the preparation to an intact skin; and which comprises a base material and an antigen or an artificial synthetic nucleic acid laminated on the surface of the base material. The regulation wherein means both the positive regulation of activating and enhancing of the immune system and a negative regulation of inhibiting of the immune system.

To explain our observed results, the transdermal immune preparation could regulate the immune response by transporting the antigen through the skin to the immune cell system without any assistance of adjuvant. The antigen permeates through the intact outlayer of the skin (eg, stratum corneum) and regulate the immune response directly or via the antigen presenting cell (eg. macrophage, dendritic cell, skin dendritic cell, B lymphocyte, or kupffer cell) which can present treated antigen in B lymphocytes. In some cases, antigen may also permeate through the stratum corneum via hair follicle or epidermal appendages (sweat glands, sebaceous glands).

When the said antigen is presented in biological immunocyte, it means the antigen is a substance which can induce specific immune response. It is better if the antigen contains either single or complex immunity epitopes which can recognize B cell receptor (the antigen located on the outer surface of B-cells) or T cell receptor.

The said antigen may be derived from the pathogens which can be infected by the organisms such as bacteria, virus, fungus and parasites, and the antigen which can be infected by cell (e.g. tumor cells or normal cells).

The said bacteria can be, for example, anthrax, campylobacter, cholera, clostridium (clostridium difficile containing), diphtheria, enterohemor-rhagic E. coli, enterotoxigenic Escherichia coli, giardia, gonococcus, helicobacter pylori or urease produced from helicobacter pylori, Influenza B Virus, Influenza Virus not classified, Legionella bacterium, meningitis bacterium, mycobacterium (containing the organisms which relates to tuberculosis), pertussis, pneumococci, Salmonella, dyscentery bacillus, staphylococcus and its enterotoxin, group A β-streptococcus hemolyticus, streptococcus B, clostridium tetani, vibro cholerae, borrelia burgdorfi, yersinia and their products.

The said virus can be, for example, adenovirus, dengue virus serotype 1∼4, ebola virus, enterovirus, hantavirus, A-E hepatitis, herpes simplex virus 1 and2, human Immunodeficiency Virus, human papillomavirus, influenza virus, measles virus, norwalk virus, Japanese equine encephalitis virus, papillomavirus, parvovirus B 19, poliovirus, rabies virus, respiratory syncytial virus, rotavirus, rubella virus, St. Louis encephalitis virus, vaccinia virus, human T-cell leukemia virus, virus vector containing the gene which can cod other antigens (eg. malaria antigen, swinepox, yellow maturity) and their products.

The said fungus may be the fungus resulted from arthrographis, sporotrichosis, aspergillosis, candidiasis etc. and other pathogenic fungus. The above parasites can be, for example, entamoeba histolytica, plasmodium, worms, schistosoma japonicum and their products.

The antigen may be the tumour related antigen or self antigens. The self antigens can be, for example, allergens of pollen, animal dandruff, aspergillus niger, dust tick, nomi antigen, saliva, grass, foods (eg. peanuts and other nuts), Betvl etc.

The antigens may be chemicals, such as carbohydrate, glycolipid, glycoprotein, lipid, lipoprotein, phospholipids, polypeptide or their chemical or recombinant compounds. The antigen may be derived by recombination, chemical synthesis or natural resources purification, preferably protein antigen or compound of antigen with polysaccharide. The antigen is at least part purified non-cell based antigen. Or it may be provided by live-virus, live attenuated virus or inactivation virus. The antigen may also be antigen encoding nucleic acid (eg. DNA, RNA, cDNA, cRNA).

The said artificial synthetic nucleic acid is the nucleic acid synthesized artificially. For example, the decoy nucleic acid, antisense nucleic acid or small interfering RNA etc.

If the molecular weight of said antigen and artificial synthetic nucleic acid is smaller than 1000 Dalton, they will be absorbed into a hydrophilic macromolecular base material when putting drops of liquid antigen on surface of the base material, making it difficult for antigen and artificial synthetic nuclei acid to be in a concentrated state on the surface of the base material. Therefore, it is desired that the molecular weights of the antigen and artificial synthetic nucleic acid are larger than 1000 Dalton.

It is desired that said base material is a hydrophilic macromolecule which can swell after absorbing water and/or water-containing alcohol. Because said antigen and artificial synthetic nucleic acid is dissolved or suspended in water and/or water-containing alcohol, when putting drops of an aqueous solution or suspension of said antigen or artificial synthetic nuclei acid on the surface of said base material, the water and/or the water-containing alcohol would be absorbed by the hydrophilic macromolecule of the base material, thereby forming an antigen or artificial synthetic nucleic acid layer in a concentrated state on the surface of the base material.

Said hydrophilic macromolecule can be, for example, poly-alkylalkoxy acrylate, starch-acrylic acid graft polymers, polyacrylates, polyvinylalcohol, vinyl acetate-acrylic acid polymers, polyvinyl pyrrolidone, isobutylene--maleic acid polymer, N-vinyl acetamides.

If said hydrophilic macromolecule is cross linked, the intensity of the macromolecule may fully be weakened after the hydrophilic macromolecule swells after water and/or water-containing alcohol are absorbed in the macromolecule. Therefore, it is desirable that the macromolecule to be crosslinked. Typical method for crosslinking can be any known method, such as including crosslinking agent like multifunctional compounds of isocyanate, metal complexes of aluminum acetylacetonate etc. into the hydrophilic macromolecule.

One of the hydrophilic macromolecules of said polyvinylalcohol which is suitable in usage is polyvinylalcohol hydrogel. Typical method for preparation of the polyvinyl alcohol hydrogel sheet can be any known method. For example, a polyvinylalcohol aqueous solution in a concentration of 5∼50% by weight is spread onto the polyethylene terephthalate film to form a layer of polyvinylalcohol aqueous solution. After being frozen at -10°C∼-2°C for I day, said aqueous solution layer is thawed to room temperature, and then a cross-linked polyvinylalcohol sheet is prepared.

It is preferable that the base material comprises a copolymer, octyldodecyl lactate, glycerin and hyaluronic acid in a concentration ratio of 100: 2∼15:2∼10:0.01∼0.1 by weight respectively. The components of said copolymer are methoxyethyl acrylate, (methyl) lauryl acrylate and polar monomer in a concentration ratio of 40∼60%:30∼40%:10∼25% by weight.

The said polar monomers are vinyl-2-pyrrolidone, (methyl) acrylic acid, 2-hydroxyehtyl acrylate ect. If the amount of methoxyethyl acrylate is increased, it will easily cause gelation and of the copolymer, thus reducing the adhesiveness. If the amount of (methyl)lauryl acrylate is decreased, the adhesiveness of the adhesive would also be reduced. Moreover, if the amount of polar monomer in base material is decreased, the cohesive force of the adhesive would be reduced. However, a larger amount of the polar monomer will also influence the adhesiveness of the adhesive. Therefore, it is preferable that methoxyethyl acrylate, (methyl) lauryl acrylate and polar monomer are in a ratio as mentioned above.

Octyldodecyl lactate, glycerin and hyaluronic acid can not only increase the hydrophlicity of said hydrophilic macromolecule, which results in higher water absorptivity of said macromolecule, but also improve its skin adhesiveness, making it easier to adhere to the skin. While a less addition of these compounds can not obtain the expected effect, an excess addition will result in an inordinate pliability of the hydrophilic macromolecule which leads to a low practicality of the macromolecule. Therefore, a concentration ratio as mentioned above is preferred.

The transdermal immune preparation in this invention comprises an antigen or an artificial synthetic nucleic acid laminated on the surface of the base material. The thickness of the base material according to this invention is not specifically limited. Too thinner base material layer would cause water and/or water-containing alcohol in the aqueous solution or suspension of the antigen or artificial synthetic nucleic acid to be insufficiently absorbed in the base material. However, too thicker base material layer will lose the pliability of the base material, thereby resulting in lower skin adhesiveness. Therefore, the thickness of the base material layer is in a range of 20∼5000µm, preferably 50∼2000µm. The type of transdermal immune preparation according to this invention is also not specifically limited. A patch preparation is desirable. Additionally, it is good to have a backing film to be laminated on surface of the base material and layer of antigen or artificial synthetic nucleic acid. Examples of the backing films are release film, polyethylene, polypropylene, polyethylene terephthalate film and urethane film.

In order to increase the absorbency of water into the base material, it is desirable to contain a plasticizer which is soluble in hydrophilic macromolecule into the base material. A hydrophilic and liquid plasticizer is preferable. Examples of the plasticizers are glycerin, ethylene glycol, polyethylene glycol, octyldodecyl lactate, sorbitan monooleate, sorbitan monopalmitate, polyoxyethylene sorbitan monooleate etc.

In order to promote the percutaneous absorption of antigen or an artificial synthetic nuclei acid, it is desirable to include chemical enhancer(s) into said base material. Said chemical enhancers can be any known chemical enhancers, for example, alcohols of menthol, camphor, cetyl alcohol etc., fatty acid esters of isopropyl palmitate, isopropyl myristate etc., glycerol esters of glyceryl monolaurate, glyceryl monooleate, etc., amides of Lauric diethanolamide, nonionic surfactants of polyethylene glycol di-n-dodecyl ether, etc. A less than necessary addition of chemical enhancer would not obtain the enhancing effect. However, an overdose of the chemical enhancer would reduce the adhesiveness of the base material. Thus, it is preferable that the base material and chemical enhancer are mixed in a ratio of 100:3∼40 by weight.

Moreover, if some enzymes as protease, esterase, lipase etc. are to be included into the base material, the percutaneous absorption of antigen or an artificial synthetic nucleic acid can be much more enhanced due to the effect of these enzymes on stratum corneum. Examples of said protease are pepsin, trypsin, chymotrypsin, papain, collagenase, elastase, endoproteinase, pronase etc. Lipase may either the refined extracts from animal pancreas or the products on the market.

Although the transdermal immune preparation in this invention is not necessary to contain any adjuvant, it is also preferable to contain the adjuvant in the base material or the layer of antigen or an artificial synthetic nucleic acid. Adjuvant is a substance which can help to induce the immune response to antigen or an artificial synthetic nuclei acid. Some adjuvants function both as adjuvants and antigen because they can induce the immune stimulation and specific antibody response or induce T cell response.

Examples of said adjuvants may be complete Freund's adjuvant or Incomplete Freund's adjuvant, chemokine{(eg. difensing) 1 or 2, RENTES, MIP1-α, MIP-2, interleukin-8 or cytokine (eg. Interleukin-1β, -2, -6, -10 or -12; Interferon y, tumor necrosis factor-α or granulocyte macrophage colony stimulating factor)}, muramyl-di-peptide (MDP) inductor (eg. murabutide, threonyl-MDP or muramyl tripeptide), heat shock protein or its inductor, inductor of leishmania major LeIF, cholera toxin cholera toxin B, bacterial ADP-ribosylating exotoxins and its subunit, bacterial ADP-ribosylating exotoxins and the recombinant using its subunit, lipopolysaccharide (LPS) inductor (eg. lipid A, monophosphoryl lipid A or structural analogs of lipid A), superantigen, ADP-ribosylating exotoxins, QS21, quill A, alum etc.

The method for producing the transdermal immune preparation is by putting drops of an aqueous solution or suspension prepared by dissolving or suspending an antigen or artificial synthetic nucleic acid in water or water-containing alcohol on the surface of a base material to cause the water or the water-containing alcohol to be absorbed in the base material, thereby forming an antigen or artificial synthetic nucleic acid layer containing the antigen or artificial synthetic nucleic acid in a concentrated state on the surface of the base material.

In this invention, the antigen or artificial synthetic nucleic acid is firstly dissolved or suspended in water or water-containing alcohol to prepare an aqueous solution or suspension. Water or water-containing alcohol can be water, water-containing methanol, water-containing ethanol etc.. Their buffer solutions may also be used. Some buffer solutions as phosphate buffer saline (PBS) containing no Ca²⁺/Mg²⁺, normal salt water (150mMNaCl water solution), or tris buffer solution etc. can be used.

Usually, antigen or artificial synthetic nucleic acid can be dissolved in water and/or water-containing alcohol or their buffer solution. If the antigen or artificial synthetic nucleic acid can not be dissolved in water and/or water-containing alcohol or their buffer solution, it may be dissolved in 10mM acetic acid at first to prepare a solution, and then diluted with neutral water or aqueous alcohol or buffer solution to expected volume. If the antigen or artificial synthetic nucleic acid can only be dissolved in acidic pH, it should be dissolved in diluted acidic acetic first to prepare a solution, and then this solution is diluted by acidic acetic acid-PBS solution to expected volume. With regard to those hydrophobic antigens or artificial synthetic nucleic acids (eg. hepatitis A virus etc.) which are self insoluble, they may also be suspended by surfactant.

Then drops of prepared aqueous solution or suspension are put on the surface of said base material to cause the water or the water-containing alcohol to be absorbed in the base material. Because said base material is a hydrophilic macromolecule which can swell after absorbing water and/or water-containing alcohol, water and/or water-containing alcohol in aqueous solution or suspension can be easily absorbed into the base material (hydrophilic macromolecule), thereby forming an antigen or artificial synthetic nucleic acid layer containing the antigen or artificial synthetic nucleic acid in a concentrated state on the surface of the base material.

The transdermal immunization method using the preparation in this invention is characterized by applying the transdermal immune preparation to an intact skin to regulate the immune response. The construction of said transdermal iuumne preparation is as mentioned above. Without striping the stratum corneum, the antigen or artificial synthetic nucleic acid can easily penetrate through or into the skin after applying the transdermal immune preparation to an intact skin.

The antigen or artificial synthetic nucleic acid penetrated through or into the skin can directly regulate the immune response, or is captured by presenting cell group (eg. macrophage or kupffer cell) and is presented in lymphocyte. In some cases, antigen or artificial synthetic nucleic acid may also permeate through the stratum corneum via hair follicle or epidermal appendages (eg. sweat glands, sebaceous glands).

For example, the transdermal immunization using bacterial ADP-ribosylating exotoxins (bARE) is on target at epidermal langerhans cell which is known as the most effective antigen presenting cell (APC). Langerhans cell can be activated by applying bARE to the lower side of the epidermal of the intact skin. Langerhans cell instructs the specific immune response by uptake of the antigen and shifting to lymph node to act as APC here to present the antigen in lymphocyte and strongly induce the antigen response.

On the other hand, the transdermal immunization using artificial synthetic nucleic acid is to prevent the discovery of the gene of inflammatory factor and restrain excess immune reaction. That is to say to proceed the negative regulation. The artificial synthetic nucleic acid permeates through the stratum corneum to restrain the discovery of the gene of immune reaction cell presenting in epidermis.

Langerhans cell, derived from marrow, is presenting in stratum spinosum layer of the epidermis of all mammals. It constitutes all the appendant cell activity presenting in uninflammatory epidermis and is necessary for initiating and propagation of the immune response to the antigen applicable in epidermis. Langerhans cell is widely distributed in epidermis and solid viscera and lymphatic system, and is the member of dendritic cell family of strong appendant cell (dendritic cell) which almost not display.

It is recognized that langerhans cell (and other dendritic cells) has at least two definite phases of live history. The langerhans cell of epidermis constitutes the normal network of antigen capture. langerhans cell can capture granule (containing microorganism) and is an effective processing substance to complex antigen. However, they can only discover the low level MHC class I and II antigen and co-stimulatory molecules (CD80, CD86 and CD86) and they are substances which can weakly stimulate the T cell not in prime shape.

After contact with antigen, part of the langerhans cells are activated, migrate from the epidermis to T cell dependent domain in local lymph nodes, and undergo maturation into activated dendritic cells. In process of migration from the epidermis to lymph nodes, shape, surface display and function of the antigen supported langerhans cell are sharply changed. Compared with epidermal langerhans cell, although dendritic cells in lymph nodes is basically not to endocytose antigens and has no efficiency in protein antigen processing, they can discover high level MHC class I and II and varies co-stimulatory molecules, and is a most effective stimulatory substance to identified natural T cell.

The strong ability of langerhans cell of epidermis to presenting antigen makes it possible to be used by transdermally delivered vaccine. The transdermal immuse response using skin immune system is, for example, passive diffusion. Then the uptake of antigens is activated by langerhans cell. These antigens are transported to lymph nodes for presenting the T cell. Then the specific immune response to the antigen (eg. BSA) is induced.

Before applying the transdermal immune preparation to the intact skin, it is desirable to pretreat the intact skin with enzyme to enhance the skin permeation of the antigen or artificial synthetic nucleic acid. The enzyme can be protease, esterase, lipase etc which are mentioned before. There is no any special limitation to the application method of the enzyme treatment. For example, the enzyme aqueous solution prepared by dissolving the enzyme in purified water of a certain pH is applied to the skin site where a transdermal administration preparation will be patched on. It is convenient in application to dip the filter paper or some other porous material into the enzyme solution and then to apply it to the skin site. As for the other pretreatment of skin with enzyme, the enzyme should be dissolved in an ointment matrix to prepare an enzymen ointment before being applied to the skin, or dissolved in hydrogel to prepare an enzyme hydrogel before being applied to the skin.

The composing of the transdermal immune preparation in this invention is as mentioned above. It can exhibit a high immunological activity without containing any adjuvant. Moreover, according to the method for producing the transdermal immune preparation in this invention, it is convenient to prepare the transdermal immune preparation comprising a concentrated antigen or artificial synthetic nucleic acid layer. Also according to the immune method in this invention, it can protect the balance of immune system without causing any inflammation, and is beneficial to therapy.

[Fig.1] Fig.1 is a bar graph of OVA specific antibody titer of examples 1 and 2, and controls 1 and 2.
[Fig.2] Fig. 2 is a bar graph of luciferase activity of examples 7 and 8, and controls 4-7.

### Effects of the invention

Next are some of the embodiments concerning this invention.

### Preparation of the base material

### (1) Base material 1

A 500ml flask was filled with 200g ethyl acetate, 43g methoxyethyl acrylate, 36g lauryl acrylate, 6g vinylpyrrolidone, 3g acrylic acid, 10g hydroxyethyl acrylate and 0.005g azobisisbutyronitrile to form a synthesize solution. The solution was copolymerized at 75°C for 15 hours in a nitrogen atmosphere.

100g of copolymer solution obtained above, 3g glycerin, 10g octyldodecyl lactate and 0.02g sodium hyaluronate were mixed completely to prepare a solution. Said solution was coated onto the PET film using a knife coater, and then dried at a temperature of 50°C for 24 hours to form a base material sheet with a thickness of 200µm. Then said sheet was punched to obtain a circular patch shaped base material about I cm in diameter.

### (2) Base material 2

40 weight% of polyvinyl alcohol was coated onto the PET film to form a sheet with a thickness of 400µm. After being frozen at -60 C° for 24 hours, the sheet was moved to room temperature to obtain the cross-linked polyvinyl alcohol hydrogel sheet. Then said sheet was punched to obtain a circular patch shaped base material about 1cm in diameter.

### (3) Base material 3

After a complete mixing of 1.5g sodium polyacrylate, 2.5g glycerin and 12.0g water, 0.1N NaOH aqueous solution and 0.1N HCl were dropped to prepare a sodium polyacrylate aqueous solution with the pH around 6. The prepared sodium polyacrylate aqueous solution was coated on the surface of the nonwoven and dried to form a base material sheet with a thickness of 400µm. Then said sheet was punched to obtain a circular patch shaped base material about Icm.in diameter

### (Example 1)

The OvalbuminGradeV (OVA, Sigma) in a molecular weight of 45,000Dalton was used as model antigen to carry out the antibody inducing experiment. 10 µl of OVA solution prepared by dissolving 10mg OVA in 1ml of phosphate buffer saline (PBS) solution which containing no Ca²⁺/Mg²⁺ was dropped on the surface of base material I in order for the PBS to be absorbed in the base material 1, thereby forming a OVA patch comprising 100 µg of OVA laminated on the surface of the base material 1.

The prepared OVA patch was applied to both sides of the ear skin of the BALB/c mouse (H-2^{d}, 7 weeks, femal, purchased from SCL Co.) for 24 hours to carry out the first immunization operation. 2 weeks later, a second immunization operation was carried out using a new OVA patch to the same site of the ear skin. 2 weeks after the second immunization operation, the blood of the mouse was collected through hematocrit capillary of eye-orbit.

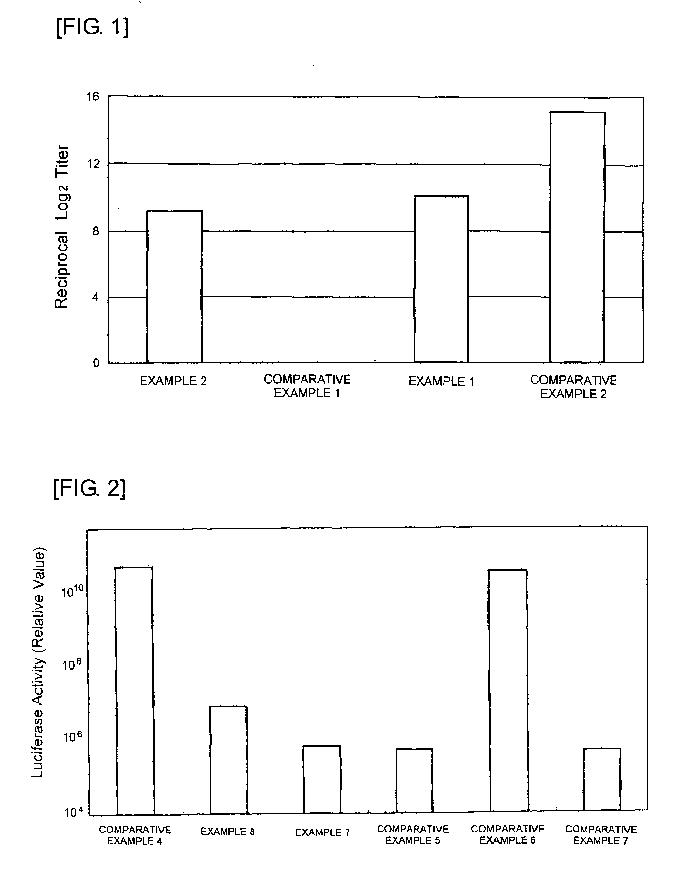

The OVA specific antibody titer in serum of the collected blood was determined by enzyme immunoassay and the result was shown in Fig. 1. Taking the highest dilution factor that the absorbance in enzyme immunoassay being larger than 0.1 as the titer, the inverse of log₂ Titer is the OVA specific antibody titer.

The enzyme immunoassay was carried out as follows:
The OVA was dissolved in a solidified buffer in a concentration of 100µg/ml, injected into the 96 well Elisa plate (Nunc Co.) in 50µl/well respectively, and then the OVA was solidified at 4°C for 1 night. Then 200µl of blocking agent (two times diluted solution of Block Ace, Dainippon Sumitomo Pharma Co., Ltd) was added into the solidified plate, and the OVA was incubated for blocking at 37°C for 2 hours. Then the serum was separated from the collected mouse blood and diluted with blocking agent with a serial of 2 times dilution. The diluted serum was added into the blocked plate in 50µl/well respectively to incubate at 37°C for 2 hours.

Then the plate was washed 3 times with buffer and added each with horseradish peroxidase (HRP) labeled goatanti-mouse IgG (5000 times diluted with blocking agent) in 50µl/well. After being incubated at 37°C for 2 hours, the plate was washed 3 times with buffer and then each added with HRP base solution in 100µl/well. After 15 minutes' incubation, 2N H₂SO₄ was added into the plate in 100µl/well to stop the reaction. Then the absorbance was determined at 450nm to evaluate the OVA specific antibody titer.

### (Example 2)

After the stratum corneum on both sides of the ear skin of the BALB/c mouse was stripped 8 times using the cellophane tape, the same immunization operation as mentioned in Example 1 was carried out. The blood was collected after the second immunization operation and the results of OVA specific antibody titer in serum was shown in Fig.1.

### (Control 1)

Except for using the patch prepared by dropping 1µl of PBS on the surface of the base material 1, the immunization operation is the same as example 2. The blood was collected after the second immunization operation and the results of OVA specific antibody titer in serum was shown in Fig.1.

### (Control 2)

A hypodermic injection of 10 µl of OVA solution prepared by dossolving 10mg OVA in 1ml PBS in BALB/c mouse ear was carried out for the first immunization operation. 2 weeks later, the mouse ear was hypodermically injected with said OVA solution to carry out the second immunization operation. 2 weeks after the second immunization operation, the blood of the mouse was collected through hematocrit capillary of eye-orbit. The OVA specific antibody titer in serum of the collected blood was determined by enzyme immunoassay and the result was shown in Fig. 1.

### (Example 3)

10 µl of OVA solution prepared by dissolving 10mg of OVA in 1ml PBS solution was dropped on the surface of the base material 2 in order for the PBS to be absorbed in the base material 2, thereby forming an OVA patch which comprising an OVA layer containing 100µg of OVA on the surface of the base material 2. The same immunization operation as mentioned in Example 1 was carried out using the prepared OVA patch. The blood was collected after the second immunization operation and the OVA specific antibody titer in serum was 9.5.

### (Example 4)

10 µl of OVA solution prepared by dissolving 10mg of OVA in 1ml PBS solution was dropped on the surface of the base material 3 in order for the PBS to be absorbed in the base material 3, thereby forming an OVA patch which comprising an OVA layer containing 100µg of OVA on the surface of the base material 3. The same immunization operation as mentioned in Example 1 was carried out using the prepared OVA patch. The blood was collected after the second immunization operation and the OVA specific antibody titer in serum was 10.5.

### (Example 5)

Both sides of the ear skin of BALB/c mouse were tightly in contact with wet gauze soaked with 0.1 weight% of trypsin (Nacalai Tesque Inc.) solution at 36 C° for I hour for pretreatment of the stratum corneum. Then the skin was washed with purified water and wiped with gauze. And then the same immunization operation as mentioned in Example 1 was carried out using OVA patch prepared in Example 1. The blood was collected after the second immunization operation and the OVA specific antibody titer in serum was 12.0.

### (Example 6)

The recombinant HBs protein solution (Sigma Co.) prepared by dissolving 40µg of recombinant HBs protein in 10µlPBS solution was dropped on the surface of the base material 2 in order for the PBS to be absorbed in the base material 2, thereby forming an recombinant HBs protein patch which comprising an recombinant HBs protein layer containing 40µg of recombinant HBs protein on the surface of the base material 2. Three white male rabbits (2∼2.5 kg) which showed negative reaction to the specific antibody to HBs were hair shaved at the center of the back, pasted with prepared patch, and then covered with dressing tape to proceed the immune sensitivity operation. The patch was removed after 24 hours. 3 weeks later, the venous blood of the rabbit was collected to determine the specific antibody titer to HBs antigen. The determination showed positive results for all three rabbits. Additionally, Bioclit-anti HBs (SankoJunyaku Co. Ltd.) was used in antibody titer determination.

### (Control 3)

The recombinant HBs protein solution (Sigma Co.) prepared by dissolving 40µg of recombinant HBs protein in 10µl PBS solution was dropped on the surface of the base material obtained by punching the gauze in a circular shape about 1cm in diameter in order for the PBS to be absorbed in the base material, thereby forming an recombinant HBs protein patch which comprising an recombinant HBs protein layer containing 40µg of recombinant HBs protein on the surface of the base material. The same operation as mentioned in Example 6 was carried out using the prepared patch to determine the specific antibody titer to HBs antigen. The determination showed negative results for all three rabbits.

### (Example 7)

Adenovirus vector was smashed to prepare the aqueous solution of adenovirus vector smash in 10mg protein/ml. 40µl of the prepared solution was dropped on the surface of the base material I to cause the PBS to be absorbed in the base material 1, thereby forming a adenovirus vector patch which comprising a adenovirus vector derived protein layer containing 100µg of said protein on the surface of the base material 1.

The same immunization operation as mentioned in Example 1 was carried out using the prepared patch. 2 weeks after the second immunization operation, adenovirus vector for finding luciferase gene (Ad-Luc) was intravenously injected in mouse tail. 48 hours after the injection, the liver of the mouse was excised to prepare the homogenate. Then the luciferase assay kit was added into the homogenate to determine the luciferase activity in liver using the microplate reader (Tecan Japan Co. Ltd., Trade name: Spectrofluoroplus). The results are shown in Fig.2

### (Control 4)

After the stratum corneum on both sides of the ear skin of the BALB/c mouse was stripped 8 times using the cellophane tape, the same immunization operation as mentioned in Example 7 was proceeded using the patch prepared by dropping PBS on the surface of the base material 1 in order for the PBS to be absorbed in the base material 1. Then the Ad-Luc was intravenously injected in mouse tail and the luciferase activity was determined by luciferase assay. The results are shown in Fig.2

### (Example 8)

After the stratum corneum on both sides of the ear skin of the BALB/c mouse was stripped 8 times using the cellophane tape, the same immunization operation as mentioned in Example 7 was carried out. Then the Ad-Luc was intravenously injected in mouse tail and the luciferase activity was determined by luciferase assay. The results are shown in Fig.2

### (Control 5)

10µl of the crushed adenovirus solution in concentration of 10mg protein/ml was hypodermically injected into the BALB/c mouse ear skin to carry out the first immunization operation. Two weeks after the second immunization operation, the Ad-Luc was intravenously injected in mouse tail as mentioned in Example 7, and the luciferase activity was determined by luciferase assay. The results are shown in Fig.2

### (Control 6)

Ad-Luc was intravenously injected in BALB/c mouse tail. 48 hours after the injection, the luciferase activity in liver was determined by luciferase assay. The results are shown in Fig.2

### (Control 7)

The luciferase activity in liver of the BALB/c mouse was determined by luciferase assay. The results are shown in Fig.2. This is a total background value determination.

### (Example 9)

50µg of the fluorescein isothiocyanate (FITC) labeled NF-κB decoy nucleic acid (HokkaidoSystem Science Co. Ltd.) aqueous solution was dropped on the surface of base material 1 to prepare a decoy nucleic acid patch comprising a FITC labeled NF-κB decoy nucleic acid layer laminated on the surface of the base material 1. The prepared decoy nucleic acid patch was applied to the back of the hairless muse. 24 hours later, the patch was removed. The skin of the hairless mouse was excised to make the skin section for observation using the fluorescence microscopy. The results showed that FITC permeated in skin deeply to 50µm from the skin surface, indicating that NF-κB decoy nucleic acid can effectivelt prevent atopic dermatitis for its deep permeation into the skin.

### (Control 8)

10µl of FITC labeld NF-κB decoy nucleic acid aqueous solution in a concentration of 5mg/ml was coated on the back of the hairless mouse. 24 hours later, the skin of the hairless mouse was excised to make the skin section for observation using the fluorescence microscopy. The results indicated that the FITC can not premeate completely into the deep skin.

### Possibility for industry production

The preparation in this invention is a non-invasive preparation which is effective for transdermal immunization, atopic therapy etc.

## Claims

1. A transdermal immune preparation which can regulate the immune response by applying the preparation to an intact skin, and which comprises a base material and antigens or artificial synthetic nucleic acids laminated on the surface of the base material.

2. The transdermal immune preparation of claim 1, wherein the antigen may be the antigen derived from the pathogen which is selected from the groups of bacteria, virus, fungus and parasites, tumor associated antigen, selfantigen, allergen, or necleic acid coded for antigen.

3. The transdermal immune preparation of claim 1, wherein the artificial synthetic nucleic acid may be the decoy nucleic acid, antisense nucleic acid or small interfering RNA (siRNA).

4. The transdermal immune preparation of claim 1, wherein the antigen or artificial synthetic nucleic acid has a molecular weight higher than 1000 Dalton.

5. The transdermal immune preparation of claim 1, wherein the base material is a hydrophilic macromolecule which can swell after absorbing water and/or water-containing alcohol.

6. The transdermal immune preparation of claim 5, wherein the hydrophilic macromolecule may be poly-alkylalkoxy acrylate, starch-acrylic acid graft polymers, polyacrylates, polyvinylalcohol, vinyl acetate-acrylic acid polymers, polyvinyl pyrrolidone, isobutylene-maleic acid polymer, N-vinyl acetamides.

7. The transdermal immune preparation of claim 6, wherein the polyvinylalcohol is polyvinylalcohol hydrogel.

8. The transdermal immune preparation of claim 5, wherein the base material is composed of a copolymer, octyldodecyl lactate, glycerin and hyaluronic acid in a concentration ratio of 100: 2∼15:2∼0:0.01∼0.1 by weight respectively.; said copolymer is copolymerized by methoxyethyl acrylate, (methyl)lauryl acrylate and polar monomer in a concentration ratio of 40∼60%:30∼40%:10∼25% by weight.

9. The transdermal immune preparation of claim 1, wherein the base material contains plasticizer, chemical enhancer and/or enzyme.

10. The said transdermal immune preparation according to claim 1 contains adjuvant.

11. The method for producing the preparations claimed in any of the claim 1 to 10, which comprises putting drops of aqueous solution or suspension prepared by dissolving or suspending an antigen or artificial synthetic nucleic acid in water or water-containing alcohol on the surface of a base material in order for the water or the water-containing alcohol to be absorbed in the base material, thereby forming an layer containing the antigen or artificial synthetic nuclei acid in a concentrated state on the surface of the base material.

12. The transdermal immunization method wherein anyone of the preparations claimed in claims 1 to 10 is applied to intact skin to regulate the immune response.

13. The transdermal immunization method according to claim 12, wherein said preparation is applied to the intact skin after said intact skin is pretreated with enzyme.
